# EUROPÄISCHE PATENTSCHRIFT

(11) **EP 2 446 016 B1**
(45) Veröffentlichungstag und Bekanntmachung des Hinweises auf die Patenterteilung: **05.12.2012**
(21) Anmeldenummer: 10740142.4
(22) Anmeldetag: 22.06.2010
(51) Int. Cl.: C12M 1/00, C12N 1/12

(54) **VERFAHREN ZUR BIOMASSEPRODUKTION UND PHOTOBIOREAKTOR ZUR KULTIVIERUNG PHOTOTROPHER ODER MIXOTROPHER ORGANISMEN ODER ZELLEN**
METHOD FOR PRODUCING BIOMASS AND PHOTOBIOREACTOR FOR CULTIVATING PHOTOTROPHIC OR MIXOTROPHIC ORGANISMS OR CELLS
PROCÉDÉ DE PRODUCTION DE BIOMASSE ET PHOTOBIORÉACTEUR POUR CULTIVER DES ORGANISMES OU DES CELLULES PHOTOTROPHES OU MIXOTROPHES

(30) Priorität: 24.06.2009 DE 102009027175
(43) Veröffentlichungstag der Anmeldung: 02.05.2012
(73) Patentinhaber: IGV Institut für Getreideverarbeitung GmbH, 14558 Bergholz-Rehbrücke (DE)
(72) Erfinder: BRONESKE, Jürgen, 14947 Nuthe-Urstromtal (DE); PULZ, Otto, 14558 Nuthetal (DE); ROTHE, Thomas, 14558 Nuthetal (DE); SCHMIDT, Karsten, 16348 Wandlitz (DE); WEIDNER, Rainer, 15711 Königs Wusterhausen (DE)
(74) Vertreter: Schubert, Klemens
(86) Internationale Anmeldenummer: PCT/DE2010/050039
(87) Internationale Veröffentlichungsnummer: WO 2010/149154

(56) Entgegenhaltungen:
- JP-A- 61 249 382
- US-A1- 2008 254 529

## Beschreibung

Die Erfindung betrifft ein Verfahren zur Produktion von Biomasse aus phototrophen oder mixotrophen Organismen oder Zellen. Ohne hierauf beschränkt zu sein, bezieht sich die Erfindung insbesondere auf die Produktion von Biomasse aus niederen Pflanzen, wie Mikroalgen oder Moosen. Ferner betrifft die Erfindung einen bei der Durchführung des Verfahrens für die Kultivierung der phototrophen oder mixotrophen Organismen oder Zellen verwendbaren Photobioreaktor.

Die Produktion von Biomasse aus phototrophen und mixotrophen Organismen oder Zellen, insbesondere aus Mikroalgen, gewinnt zunehmend an Bedeutung. Die dabei erzeugte Biomasse wird inzwischen für unterschiedlichste Zwecke eingesetzt. Sie dient beispielsweise der Herstellung ernährungsphysiologisch hochwertiger Nahrungs- und Nahrungsergänzungsmittel, als Zusatzstoff für dermatologische Medikamente oder kosmetische Erzeugnisse oder auch zur Herstellung von Energieträgern. Zentrale Bestandteile entsprechender Anlagen zur Biomasseproduktion sind Bioreaktoren zur Kultivierung der Organismen oder Zellen. Während entsprechende Bioreaktoren in der Vergangenheit zumeist nach Labormaßstäben und somit mit verhältnismäßig geringer Produktionskapazität ausgelegt wurden, wird heute die Errichtung von Einrichtungen zur Großproduktion von Biomasse forciert.
In Bezug auf die Herstellung von Biomasse auf der Grundlage phototropher oder mixotropher Organismen oder Zellen besteht dabei das Problem, Photobioreaktoren bereitzustellen, welche einerseits ein großes Volumen zur Kultivierung der Organismen beziehungsweise Zellen bereitstellen und bei denen andererseits trotz der im Zuge der Kultivierung innerhalb des Reaktors anfallenden großen Mengen von Biomasse eine ausreichende und gleichmäßige Versorgung der zu kultivierenden Organismen beziehungsweise Zellen mit den dazu erforderlichen Nährstoffen und mit Licht gewährleistet ist.

Eine bekannte Möglichkeit der Biomasseproduktion besteht in der Verwendung von Rohrbioreaktoren, bei denen eine Suspension mit den Organismen oder Zellen durch in einem lichtdurchfluteten Reaktorraum angeordnete Glasrohre geführt wird. Zwar werden mittels entsprechender Glasrohrreaktoren im Hinblick auf die Versorgung der Organismen mit Nährstoffen und Licht durchaus gute Kultivierungsbedingungen bereitgestellt, jedoch sind solche Reaktoren für die Massenproduktion von Biomasse, insbesondere unter Kostengesichtspunkten, eher ungeeignet.

Ferner ist es bekannt, eine Suspension mit Organismen oder Zellen zur Versorgung mit Nährstoffen und Licht derart in einem Umlauf zu halten, dass ständig ein Teil der Suspension aus einem Reservoir am Boden eines kesselförmigen Bioreaktors beziehungsweise Photobioreaktors abgepumpt und oberhalb des Reservoirs in den mit Nährstoffen und Licht durchsetzten Reaktorraum hinein zerstäubt wird und schließlich die bei der Zerstäubung entstandenen Tröpfchen der Suspension aufgrund der Schwerkraft wieder in das Reservoir herabrieseln. Während ihrer Abwärtsbewegung von einem der Zerstäubung dienenden Ausbringungsorgan zum Reservoir am Boden des Reaktors nehmen dabei die Organismen oder Zellen in den Tröpfchen gasförmige in den Reaktorraum eingebrachte Nährstoffe und den Reaktorraum durchflutendes oder in ihn mittels künstlicher Beleuchtung eingebrachtes Licht auf. Ein solches Konzept wird beispielsweise in der US 2008/0254529 A1 beschrieben. Gemäß der in der genannten Schrift offenbarten Lösung werden die gasförmigen Nährstoffe im Bereich der Oberseite des Reaktorraums eingetragen und die Suspension über eine in einem mittleren Bereich angeordnete Düse zerstäubt.

Eine Verbesserung dieses Konzeptsbesteht darin, die in einem Umlauf geführte Suspension mit Organismen oder Zellen im oberen Bereich eines Kulturraums des Photobioreaktors über geeignete Ausbringungsorgane (zum Beispiel Sprühdüsen) auszubringen und die sich danach aufgrund des Wirkens der Schwerkraft vollziehende Abwärtsbewegung der Suspension durch die Anordnung geeigneter Innenraumelemente beziehungsweise Einbauten in dem Kulturraum zu verzögern, damit eine intensive Exposition der Organismen oder Zellen mit in den Reaktor eingebrachten Nährstoffen und Licht gewährleistet ist. Entsprechend bekannter Lösungen handelt es sich bei den vorgenannten Innenraumelementen beispielsweise um eine Mehrzahl parallel zueinander angeordneter, sich vertikal erstreckender Gewebebahnen, so genannter Matrizes beziehungsweise Sheets. An den in der Regel aus einem hydrophilen Material bestehenden Gewebebahnen werden die Organismen beziehungsweise Zellen der oberhalb dieser Gewebebahnen ausgebrachten Suspension zumindest teilweise immobilisiert. Sie sind auf diese Weise für längere Zeit dem unmittelbar im Reaktor erzeugten oder ihm zugeführten Licht und den Nährstoffen intensiv ausgesetzt. Allerdings wird mit zunehmender Anlagerung von Biomasse an den Gewebebahnen der Lichtfluss durch den Reaktor beziehungsweise dessen Kulturraum zunehmend beeinträchtigt. Insbesondere die nicht in den Außenbereichen angeordneten Materialbahnen werden dabei zunehmend verschattet, so dass für die an ihnen abgelagerte Biomasse keine optimalen Kultivierungsbedingungen mehr bestehen. Zudem gestaltet sich bei Bioreaktoren, in denen die Organismen oder Zellen in der zuvor beschriebenen Weise an entsprechenden Materialbahnen immobilisiert werden, die Ernte der Biomasse nach der Beendigung des Kultivierungsvorgangs vergleichsweise aufwändig. Dabei wird die entsprechende Biomasse beispielsweise mittels Hochdruckreinigern von den Materialbahnen abgespült. Dies ist zeitintensiv und erfordert im Allgemeinen bei der Ernte den Einsatz menschlicher Arbeitskraft.

Aufgabe der Erfindung ist es, eine alternative Lösung für die Großproduktion von Biomasse anzugeben, welche so gestaltet ist, dass sie insbesondere auch bei der Kultivierung sehr großer Mengen von Biomasse die Bereitstellung günstiger Kultivierungsbedingungen ermöglicht und dennoch einen vergleichsweise einfachen Aufbau entsprechender Produktionsanlagen zulässt. Hierzu sind ein Verfahren anzugeben und ein Photobioreaktor zur Kultivierung phototropher oder mixotropher Organismen oder Zellen bereitzustellen.

Die Aufgabe wird durch ein Verfahren mit den Merkmalen des Hauptanspruchs gelöst. Ein die Aufgabe lösender Photobioreaktor ist durch den ersten Sachanspruch charakterisiert. Vorteilhafte Aus- beziehungsweise Weiterbildungen der Erfindung sind durch die jeweiligen Unteransprüche gegeben.
Im Gegensatz zu autotrophen, sich selbst aus anorganischen Stoffen ernährenden Organismen ernähren und vermehren sich phototrophe Organismen oder Zellen unter Einwirkung von Energie, insbesondere Licht, und durch Assimilation von insbesondere Kohlendioxid. Die mixotrophen Organismen stellen entsprechend ihrem Namen eine Mischform dar, welche neben Kohlendioxid auch organische Stoffe assimilieren können und dabei im Allgemeinen Photosynthese betreiben. Das Verfahren betrifft demnach die Produktion von Biomasse aus Organismen oder Zellen, deren Wachstum und Vermehrung in jedem Falle im Wege der Photosynthese und unter Versorgung mit Nährstoffen, wie insbesondere Kohlendioxid erfolgt. Das Verfahren dient vorzugsweise der Produktion von Biomasse aus phototrophen oder mixotrophen Mikroalgen ohne jedoch, wie eingangs bereits ausgeführt, hierauf beschränkt zu sein.

Nach dem zur Produktion von Biomasse aus phototrophen oder mixotrophen Organismen oder Zellen vorgeschlagenen Verfahren werden die Organismen oder Zellen in einer in einem Photobioreaktor in Umlauf gehaltenen Suspension kultiviert. Bei der Kultivierung werden den Organismen oder Zellen während des Umlaufs der Suspension Energie in Form natürlichen oder künstlichen Lichts und mindestens CO₂ als gasförmiger Nährstoff zugeführt. Hierzu wird die Suspension über mindestens ein Ausbringungsorgan in einem oberen Bereich eines Kulturraums des Photobioreaktors ausgebracht. Die sich durch das Wirken der Schwerkraft vollziehende Abwärtsbewegung der ausgebrachten Suspension in dem Kulturraum wird durch mindestens ein geeignetes, in dem Kulturraum angeordnetes Innenraumelement verzögert, damit eine intensive Exposition der Organismen oder Zellen mit in den Kulturraum eintretenden oder darin erzeugten Licht und mit dem oder den gasförmigen Nährstoffen gewährleistet ist. Die am Boden des Kulturraums aufgefangene Suspension wird schließlich zur Realisierung des Umlaufs mittels eines Pumpsystems erneut dem mindestens einen Ausbringungsorgan zugeführt. Nach Beendigung der Kultivierung der Organismen oder Zellen wird die Biomasse geerntet, indem sie von der Suspension getrennt wird. Die geschieht mit Hilfe entsprechender Mittel zur Abtrennung, wie Separatoren, Filtern oder Siebeinrichtungen.
Erfindungsgemäß wird die Suspension für die schon angesprochene Verzögerung ihrer Abwärtsbewegung in dem Kulturraum in eine Vielzahl von Tropfen überführt. Die Suspension wird dabei an einer dafür ausgebildeten Struktur eines oder mehrerer Innenraumelemente des Kulturraums zur Ausbildung der Tropfen veranlasst. Die in Tropfen überführten Anteile der Suspension durchlaufen erfindungsgemäß während des Passierens des Kulturraums bezogen auf einen einzelnen Tropfen jeweils zumindest einmalig einen sich wie folgt vollziehenden Tropfenzyklus:
● Bildung des Tropfens an der dafür im Kulturraum vorgesehenen Struktur,
● Allmähliche Vergrößerung des Tropfens bis auf eine maximale Größe, während welcher die in dem Tropfen enthaltenen Organismen oder Zellen dem Licht und dem oder den gasförmigen Nährstoffen ausgesetzt sind und sich vermehren,
● Nach dem Erreichen einer Maximalgröße Abtropfen des Tropfens in den Auffangbereich am Boden des Kulturraums oder auf eine weitere, Tropfen erzeugende Struktur eines Innenraumelements des Kulturraums.

Durch die Überführung der Suspension in eine Vielzahl von Tropfen werden viele kleine, jeweils eine Grenzfläche zwischen Flüssigkeit (Suspension mit Organismen oder Zellen) und Gas (Atmosphäre des Reaktorinnenraums mit darin enthaltenen gasförmigen Nährstoffen) ausbildende Oberflächen geschaffen, welche in Summe eine sehr große Oberfläche beziehungsweise Grenzfläche ausbilden, an der eine besonders intensive Exposition der Organismen oder Zellen mit dem Licht und den Nährstoffen erfolgt. Durch die kleinvolumigen Tropfen minimiert sich außerdem die mittlere Weglänge der Organismen oder Zellen zur Grenzoberfläche. Während der Phase der Vergrößerung des Tropfens kann dieser unterschiedliche Formen annehmen, wobei eine mögliche Form die fluiddynamisch typische Tropfenform ist, welche an der Unterseite annähernd kreisrund ist und an der Oberseite spitz zuläuft. Welche Form der Tropfen jeweils annimmt, hängt dabei insbesondere von der Beschaffenheit der Suspension einerseits sowie von der Ausbildung der die Tropfenbildung bewirkenden Innenraumelemente andererseits ab. Die Form lässt sich aber im gewissen Maße auch durch die Art der Ausbringung der Suspension über das mindestes eine Ausbringungsorgan steuern. Wie noch ausgeführt werden soll, sind dabei vorzugsweise bestimmte Tropfenformen anzustreben.
Es hat sich zudem gezeigt, dass sich in den Tropfen aufgrund der Lichteinwirkung und der damit einhergehenden Erwärmung sowie aufgrund von durch Konzentrationsunterschiede verursachten Stofftransporten Turbulenzen einstellen, so dass die Organismen oder Zellen wiederholt an der Grenzoberfläche verweilen, wo eine besonders starke Exposition mit dem Licht und den Nährstoffen erfolgt. Bei Versuchen haben sich insoweit gute Kultivierungsergebnisse eingestellt. Es wird daher davon ausgegangen, dass die Verzögerung der Abwärtsbewegung der Suspension in dem Kulturraum, welche vorteilhafterweise ohne Immobilisierung der Biomasse erreicht wird, und die in den Tropfen ablaufenden dynamischen Prozesse das Kultivierungsergebnis vorteilhaft beeinflussen.
Bezüglich der bereits angesprochenen Tropfenform hat sich eine linsenförmige Tropfenform als sehr vorteilhaft erwiesen, da hierdurch ein verbesserter beziehungsweise sehr konzentrierter Lichteintritt in den Tropfen und damit insbesondere bei Schwachlichtverhältnissen eine sehr gute Versorgung der Organismen beziehungsweise Zellen mit der Lichtenergie erreicht wird. Gemäß einer vorteilhaften Verfahrensgestaltung wird daher die Suspension durch eine entsprechende Ausbildung der Innenraumelemente und/oder eine entsprechende Steuerung ihrer Ausbringung über das mindestens eine Ausbringungsorgan zur Bildung überwiegend linsenförmiger Tropfen veranlasst.
Als vorteilhaft hat sich aber auch die Erzeugung nebelartiger Tropfen, das heißt kugelförmiger Tropfen im Mikrobereich erwiesen. Hierbei erfolgt eine optimale Wechselwirkung zwischen Organismen beziehungsweise Zellen und den Photonen im gesamten Kulturraum beziehungsweise Kultivierungsraum. Entsprechend einer möglichen Verfahrensgestaltung werden zur Ernte der Biomasse die an den dafür vorgesehenen Strukturen des Kulturraums ausgebildeten Tropfen von diesen Strukturen durch Schwingungseinwirkung oder durch stoßartige Erschütterungen gelöst. Die sich am Boden des Kulturraums sammelnde Suspension mit den darin enthaltenen Organismen oder Zellen wird dann, wie bereits ausgeführt, Mitteln zur Abtrennung der Organismen oder Zellen aus der Suspension zugeleitet. Entsprechend einer möglichen Variante dieser Verfahrensgestaltung werden die Tropfen von den Strukturen des Kulturraumes mittels auf die Strukturen einwirkenden Ultraschalls gelöst.
Gemäß einer anderen Verfahrensgestaltung erfolgt das Lösen der Tropfen von den Strukturen des Kulturraums dadurch, dass die Tropfen mittels eines Gebläses abgeblasen werden. Die sich am Boden sammelnde Suspension wird dann ebenfalls wiederum Mitteln zur Abtrennung der Organismen oder Zellen aus der Suspension zugeführt. Schließlich besteht eine weitere Möglichkeit der Ernte der erzeugten Biomasse darin, dass die Tropfen der Suspension, welche sich an den dafür vorgesehenen Strukturen des Kulturraums ausgebildet haben, mit Hilfe einer Spülflüssigkeit abgespült werden. Dabei wird die entsprechende Spülflüssigkeit anstelle der Suspension über das mindestens eine Ausbringungsorgan im Kulturraum ausgebracht. Demzufolge ist hierfür lediglich ein Umschalten der Leitungswege des vorhandenen Pumpsystems erforderlich.
Unabhängig von der zum Ablösen der Tropfen von den Strukturen des Kulturraums gewählten Vorgehensweise ist es entsprechend einer vorteilhaften Ausgestaltung des Verfahrens vorgesehen, an den Innenraumelementen des Kulturraums verbliebene Filme der die Organismen oder Zellen aufnehmenden Suspension durch Nachspülen mit einer Spülflüssigkeit zu entfernen. Sowohl für das eventuell mittels einer Spülflüssigkeit erfolgende Lösen der Tropfen von den Strukturen der Innenraumelemente als auch für das gegebenenfalls erfolgende Nachspülen zum Zwecke des Entfernens verbliebener Suspensionsfilme wird zum Beispiel Wasser oder Nährlösung als Spülflüssigkeit verwendet.
Der die Aufgabe lösende und bei der Durchführung des Verfahrens für die Kultivierung verwendbare Photobioreaktor besteht aus
● einem Kulturraum, welcher von natürlichem Licht oder von außerhalb oder innerhalb des Kulturraums erzeugtem künstlichen Licht durchsetzt ist und in welchem mindestens CO₂ als gasförmiger Nährstoff eingebracht wird,
● mindestens einem Ausbringungsorgan, über welches die in der Suspension enthaltenen Organismen oder Zellen zur Exposition mit dem Licht und dem oder den gasförmigen Nährstoffen in einem oberen Bereich des Kulturraums ausgebracht werden
● mindestens einem im Kulturraum angeordneten Innenraumelement, durch welches die sich aufgrund des Wirkens der Schwerkraft vollziehende Abwärtsbewegung der Suspension in dem Kulturraum verzögert wird sowie
● einem Pumpensystem, durch welches die am Boden des Kulturraums aufgefangene Suspension zur Realisierung eines Umlaufs erneut dem mindestens einen Ausbringungsorgan zugeführt wird.

Erfindungsgemäß ist in dem Kulturraum des Photobioreaktors unterhalb des mindestens einen Ausbringungsorgans mindestens ein Innenraumelement mit einer sich horizontal erstreckenden Gitter-, Sieb- oder Netzstruktur angeordnet. Durch die vorgenannte erfindungsgemäße Struktur wird die in den Kulturraum eingebrachte Suspension mit den Organismen oder Zellen zur Ausbildung einer Vielzahl von Tropfen veranlasst, welche sich nach ihrer Entstehung vergrößern und jeweils nach dem Erreichen einer maximalen Tropfengröße in den Auffangbereich am Boden des Kulturraums oder auf eine weitere im Kulturraum unterhalb der vorgenannten Struktur angeordnete, Tropfen erzeugende Struktur eines Innenraumelements vergleichbarer Art abtropfen.
Die erfindungsgemäße, in dem Kulturraum horizontal angeordnete Struktur kann unter Verwendung unterschiedlichen Materials realisiert werden und hinsichtlich ihrer Geometrie verschiedenartig gestaltet sein. Demgemäß handelt es sich beispielsweise bei Verwendung flexibler Materialien um eine eher netzartige Struktur, während mittels starrer Materialien Gitter- oder Siebstrukturen realisiert werden, wobei sich Letztere nur geringfügig hinsichtlich ihrer geometrischen Ausbildung unterscheiden, so dass man eine entsprechende Struktur gegebenenfalls als Gitterstruktur oder als Siebstruktur bezeichnen kann. In jedem Falle haben aber alle genannten Strukturen (Gitter-, Sieb- oder Netzstruktur) die gleiche Wirkung beziehungsweise sind zur Erreichung des gleichen Ziels ausgelegt, nämlich zur Überführung der in den Kulturraum eintretenden Suspension in eine Vielzahl von Tropfen. Im Weiteren soll daher vereinfachend und verallgemeinert von einer Gitterstruktur gesprochen werden, wobei sich die entsprechenden Darstellungen gleichermaßen auf Netz- oder Siebstrukturen beziehen.
Je nach der Beschaffenheit der Suspension und der Gittergeometrie, das heißt der Abstände zwischen den Gitterstegen und Gitterknoten, entwickeln sich die erfindungsgemäß erzeugten Tropfen jeweils entweder an einem Gitterknoten, einem Gittersteg oder auch, unter Überbrückung der jeweils angrenzenden Gitterstege, in einem Gitterfenster. Entscheidend ist dabei jedoch nur, dass durch die Tropfenbildung die Vertikalbewegung des Suspension mit den Organismen oder den Zellen im Kulturraum zeitlich verzögert wird und sich hierbei der bei den Ausführungen zum Verfahren erläuterte Tropfenzyklus vollzieht, welcher die Exposition der Organismen oder Zellen mit dem Licht und den Nährstoffen begünstigt. Die mindestens eine Gitterstruktur beziehungsweise gitterartige Struktur (Sieb- oder Netzstruktur) besteht entsprechend einer bevorzugten Ausbildungsform des erfindungsgemäßen Photobioreaktors aus einem hydrophoben Material. Dabei wird angenommen, dass die Verwendung hydrophober Materialien insoweit vorteilhaft ist, als sich hierdurch im Grunde keinerlei Immobilisierungsvorgänge vollziehen. Auch die Anteile an den Reaktorwänden beziehungsweise den Wänden des Kulturraums und den Innenraumelementen als Film verbleibender Suspension sollten hierdurch geringer sein, so dass sich der Aufwand für ein eventuelles Nachspülen nach der Kultivierung verringert.. Das Material sollte zudem vorzugsweise so gewählt werden, dass sich an den Gitterstegen beziehungsweise den Netzmaschen oder den Zwischenräumen zwischen den Sieblöchern beziehungsweise -fenstern hydraulisch glatte Oberflächen ausbilden. Auch hierdurch wird die Filmbildung minimiert. Darüber hinaus ist die Verwendung transparenter Materialien für die Gitterstruktur als vorteilhaft anzusehen, da hierdurch eine gleichmäßige Durchflutung des Kulturraums mit dem Licht am wenigsten behindert wird und eine ausreichende Versorgung der Organismen beziehungsweise Zellen mit dem Licht eine wichtige Voraussetzung für den Kultivierungserfolg ist. Besonders vorteilhaft sind insoweit auch lichtleitende Materialien für die Realisierung der Gitterstruktur. Aber auch die Verwendung weißer Materialien hat sich als praktikabel erwiesen.
An dieser Stelle sei erwähnt, dass die entsprechend dem erfindungsgemäßen Verfahren vorgesehene Überführung der ausgebrachten Suspension in eine Vielzahl von Tropfen gegebenenfalls auch mit ähnlichen Strukturen an vertikal oder geneigt gegen die Horizontale innerhalb eines Bioreaktors angeordneten Innenraumelementen erfolgen kann. Beispielsweise erscheinen auch Tropfen bildende Strukturen beziehungsweise Oberflächenstrukturen an pyramidenartigen Elementen oder an Innenraumelementen in "Tannenbaumform" grundsätzlich denkbar. Dies lässt das beanspruchte Verfahren ausdrücklich offen, so dass dieses insoweit gegebenenfalls auch unabhängig von der für die Gestaltung eines Photobioreaktors unter Schutz gestellten Lösung ausführbar ist. Bezüglich der beanspruchten Vorrichtung bezieht sich die Erfindung jedoch, wie vorstehend ausgeführt und beansprucht, auf einen als praktikabel angesehenen und sich bezüglich der Anordnung der die abwärts gerichtete Bewegung der Suspension verzögernden Innenraumelemente klar vom Stand der Technik unterscheidenden Photobioreaktor, in dessen Kulturraum entsprechende Innenraumelemente horizontal angeordnet sind. Dabei haben in diesem Sinne sicherlich auch Innenraumelemente, die insbesondere auf Grund von Toleranzen ihrer Geometrie und der zu ihrer Anordnung sowie Befestigung dienenden Mittel, geringfügige Neigungen aufweisen, als horizontal angeordnet zu gelten.
Eine bevorzugte Ausbildungsform des erfindungsgemäßen Photobioreaktors ist dadurch gegeben, dass in dem Kulturraum mehrere sich horizontal erstreckende Gitter-, Sieb- oder Netzstrukturen in einer Kaskade untereinander angeordnet sind. Die Gitterfenster, die Netzmaschen beziehungsweise die Durchbrüche der einzelnen zueinander parallelen Gitterstrukturen beziehungsweise gitterähnlichen Strukturen aber auch die Gitter selbst können dabei in Abhängigkeit von der Geometrie des Kulturraums und/oder der Beschaffenheit der Suspension durchaus unterschiedlich groß sein, wobei es insbesondere von der Art der Suspension sowie von den jeweils gegebenen und im Falle der Nutzung natürlichen Lichts vom Einsatzort zu Einsatzort variierenden Lichtverhältnissen abhängt, ob die Gitterfenster beziehungsweise Maschen oder Durchbrüche in der vertikalen Bewegungsrichtung der Suspension größer oder kleiner werden.
Bei einer weiterhin vorgesehenen Ausbildungsform wird ein Abtropfspeicher dadurch realisiert, dass in dem Kulturraum mehrere sich von der einen vorhandenen horizontalen Gitterstruktur oder - im Falle mehrerer, parallel angeordneter Gitterstrukturen - sich von der letzten horizontalen Gitterstruktur vertikal nach unten erstreckende Netze, Schnüre, Bänder oder Ketten angeordnet sind. Die von der betreffenden Gitterstruktur abtropfenden Tropfen der Suspension laufen hierbei über diese Netze, Schnüre, Bänder oder Ketten in Richtung des Bodens des Kulturraums nach unten ab. Hierbei hat es sich gezeigt, dass für einen mittels der vorgenannten Anordnungen realisierten Abtropfspeicher die Verwendung hydrophiler Materialen vorteilhaft ist.
Der Photobioreaktor kann noch dadurch weitergebildet sein, dass dieser eine Einheit zur Erzeugung stoßartiger Erschütterungen zum Lösen der nach der Beendigung des Umlaufs der Suspension an der mindestens einen Gitterstruktur verbliebenen Tropfen aufweist. Bei einer anderen, ebenfalls vorteilhaften Ausbildungsform weist der Photobioreaktor für die Ernte der Biomasse einen Ultraschallsender auf, dessen Ultraschallschwingungen nach der Beendigung des Umlaufs der Suspension zum Lösen der an der Gitterstruktur verbliebenen Tropfen auf die entsprechende Gitterstruktur einwirken. Bei einer weiteren möglichen Ausbildungsform ist zum Lösen der an der Gitterstruktur nach der Kultivierung verbliebenen Tropfen und damit zur Unterstützung des Erntevorgangs ein Gebläse in dem Photobioreaktor beziehungsweise in dessen Kulturraum angeordnet.

Die Erfindung soll nachfolgend anhand von Zeichnungen und Ausführungsbeispielen nochmals erläutert werden, wobei die Erfindung im Folgenden beispielhaft in Bezug auf die Kultivierung phototropher Mikroalgen dargestellt wird. Die dargestellten Abläufe stellen sich aber in gleicher beziehungsweise grundsätzlich vergleichbarer Weise bei der Kultivierung anderer phototropher oder mixotropher Organismen oder Zellen ein. In den zugehörigen Zeichnungen zeigen:
- Fig. 1:: die schematische Darstellung zweier Möglichkeiten der Einordnung erfindungsgemäßer Innenraumelemente in dem Kulturraum eines Photobioreaktors,
- Fig.2:: den sich erfindungsgemäß einstellenden Tropfenzyklus in einer schematischen Darstellung,
- Fig. 3:: die Einzelheit X der Fig. 1 in räumlicher Darstellung,
- Fig. 4:: die mögliche Ausbildung des Kulturraums eines erfindungsgemäßen Photobioreaktors in einer schematischen Darstellung.

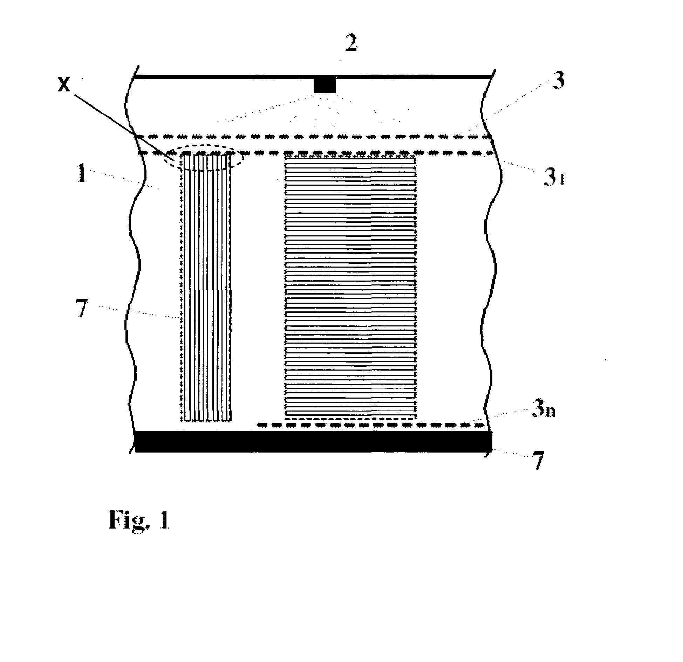

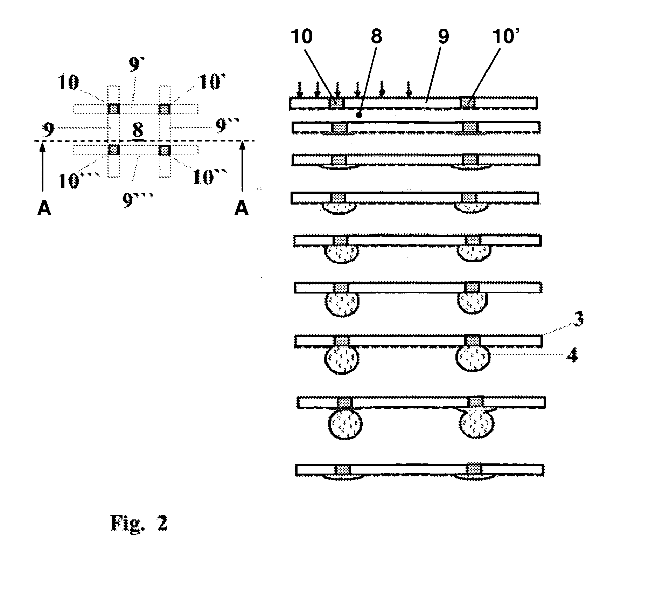

Die Fig. 1 zeigt zwei Beispiele möglicher Gestaltungen eines erfindungsgemäßen Bioreaktors mit Innenraumelementen 3, 3₁, 3ₙ ,7 in einer schematischen Darstellung. Auf der rechten Seite der Abbildung ist eine Ausbildungsvariante dargestellt, bei der mehrere Innenraumelemente 3, 3₁, 3ₙ mit Gitter- oder Siebstrukturen horizontal, parallel zueinander angeordnet sind. Bei dieser Anordnung durchlaufen die in Tropfenform überführten Anteile der Suspension mit den Mikroalgen den nachfolgend anhand der Fig. 2 erläuterten Tropfenzyklus mehrmals. Die linke Seite hingegen betrifft eine mögliche Ausbildungsvariante, bei der an der Unterseite eines die Suspension in eine Vielzahl von Tropfen 4 überführenden Innenraumelements 3₁ mit einer Gitterstruktur mehrere Bänder 7 vertikal angeordnet sind. Hierbei laufen die Tropfen 4 der Suspension nach dem Abtropfen von der Gitterstruktur des Innenraumelements 3₁ über die vertikale, insoweit als Abtropfspeicher wirkende Struktur der Bänder 7 nach unten. Bei beiden in der Fig. 1 gezeigten Varianten wird die Suspension schließlich am Boden beziehungsweise in einem Auffangbereich 6 des Kulturraums 1 aufgefangen und erneut dem oberhalb der Gitterstruktur angeordneten Ausbringungsorgan 2 zugeführt. Bei Letzterem handelt es sich beispielsweise um eine Sprühdüse.
In der Fig. 2 ist beispielhaft der Ablauf des sich an einer Gitterstruktur einstellenden Tropfenzyklus dargestellt, wobei auf der linken Seite die Draufsicht auf eine Gittermasche mit einem Gitterfenster 8, Gitterstegen 9, 9', 9", 9"' und Gitterknoten 10, 10', 10", 10"' gezeigt ist und die rechte Seite jeweils den Teil der entsprechenden Gitterstruktur eines Innenraumelements 3 in einer Schnittdarstellung mit einem Schnitt entlang der Linie A-A bezogen auf die links stellvertretend dargestellte einzelne Gittermasche zeigt. Die oberhalb der Gitterstruktur über das Ausbringungsorgan 2, beispielsweise eine Sprühdüse verrieselte Suspension mit den Organismen (beispielsweise Mikroalgen) durchdringt die Gitterfenster 8 und bildet zunächst unterhalb der jeweiligen Masche einer Gitterstruktur jeweils bereichsweise ein dünne, filmartige Schicht an den Gitterknoten 10, 10', 10", 10"' und Gitterstegen 9, 9', 9", 9"' aus. Durch weitere nachlaufende Anteile der Suspension entsteht schließlich im Bereich der Gitterknoten 10, 10', 10", 10"' ein Tropfenansatz. Dieser entwickelt sich zu einem sich danach allmählich vergrößernden Tropfen 4. Innerhalb des Tropfens 4 entstehen durch das auf den Tropfen 4 auftreffende Licht und die damit einhergehende Erwärmung sowie durch tropfeninterne Stofftransporte Turbulenzen, in deren Folge die in der Suspension enthaltenen Mikroalgen wiederholt an die Tropfenoberfläche gelangen, wo sie im Allgemeinen eine gewisse Zeit verweilen und dabei besonders gut Energie in Form des Lichtes und Nährstoffe (insbesondere CO₂) aus der gasförmigen Umgebung im Kulturraum aufnehmen. Der Tropfen 4 wächst dann zu einer von seiner jeweiligen Oberflächenspannung abhängigen maximalen Größe heran und es bildet sich an seiner Oberseite im Übergangsbereich zu der Gitterstruktur eine Einschnürung. Schließlich tropft der Tropfen 4 ab, wobei er entweder auf eine weitere Gitterstruktur eines Innenraumelements 3, 3₁, 3ₙ auftrifft oder sich in Richtung des Auffangbereichs 6 am Boden des Kulturraums 1 bewegt. Letzteres kann, je nach Ausgestaltung des Photobioreaktors, gegebenenfalls auch dadurch geschehen, dass der Tropfen 4 an dafür unterhalb der Gitterstruktur angeordneten Bändern, Schnüren oder Ketten oder vertikalen Netzen 7 hinunterläuft.

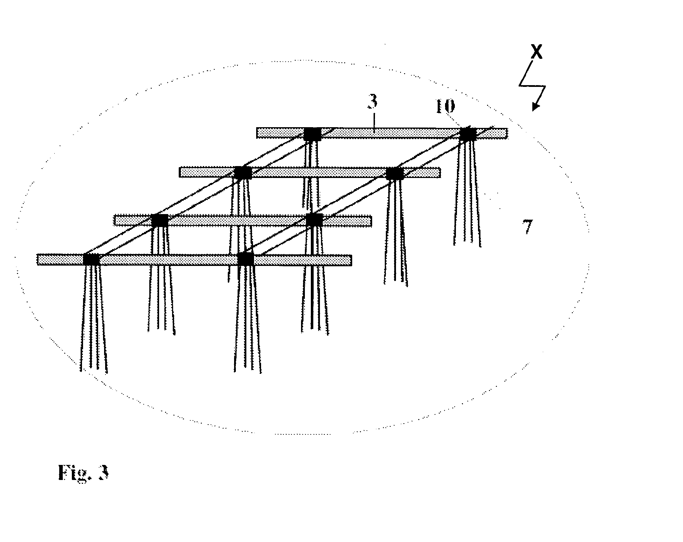

Die Fig. 3 betrifft in vergrößerter Darstellung die Einzelheit X der Fig. 1, nach welcher unterhalb einer Gitterstruktur entsprechende vorgenannte Bänder, Schnüre oder dergleichen angeordnet sind, in einer räumlichen Darstellung. Es handelt sich hierbei beispielsweise um Ultradünnschichtbänder, über welche die Tropfen 4 unter Ausbildung dünner Schichten auf den Bändern 7 von den Gitterknoten 10, 10', 10", 10"' der Gitterstruktur eines Innenraumelements 3, 3₁, 3ₙ nach unten zum Boden des Kulturraums 1 abgeleitet werden. Die entsprechenden Bänder 7 sind vorzugsweise zumindest leicht hydrophil, so dass es zu einer teilweisen Immobilisierung der Biomasse kommt. Die entsprechenden Ablagerungen werden dann vorzugsweise im Zusammenhang mit der Ernte der Biomasse von den Bändern 7 abgespült.

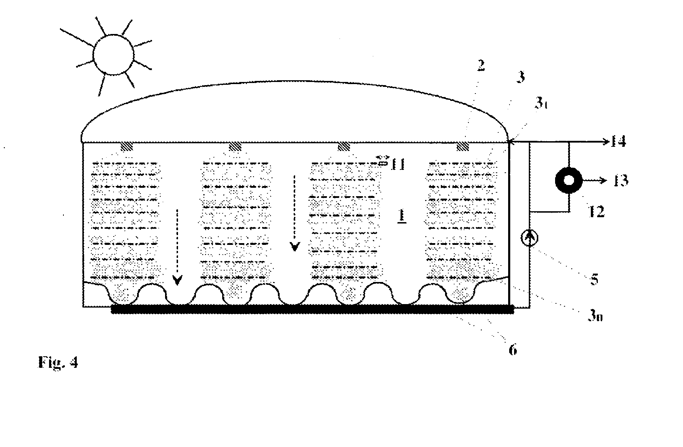

In der Fig. 4 ist nochmals eine mögliche Ausbildungsform des Kulturraums 1 eines erfindungsgemäßen Photobioreaktors in schematischer Darstellung gezeigt, wobei zur Versorgung der zu kultivierenden phototrophen Mikroalgen mit Licht das natürliche Sonnenlicht genutzt wird, welches den Kulturraum 1 des Photobioreaktors durchsetzt, wobei der Kulturraum 1 des beispielhaft gezeigten Photobioreaktors zu diesem Zweck mit transparenten Wänden ausgebildet ist. Wie aus der Figur erkennbar, wird die Suspension bei dieser Ausbildungsform über eine Mehrzahl von Ausbringungsorganen 2 ausgebracht. Unterhalb dieser Ausbringungsorgane 2 sind in dem Kulturraum 1 jeweils kaskadenartig mehrere horizontale Innenraumelemente 3, 3₁, 3ₙ mit Gitterstrukturen angeordnet. An ihnen vollzieht sich jeweils mehrfach der gemäß Fig. 2 erläuterte Tropfenzyklus. Unterhalb dieser Gitterkaskaden sind kanalartige Auffangbereiche 6 vorgesehen, über welche die abtropfende Suspension abgeleitet und schließlich mittels eines nicht in seinen Einzelheiten gezeigten Pumpsystems 5 erneut den Ausbringungsorganen 2 zugeführt wird.

### Liste der Bezugszeichen

- 1: Kulturraum
- 2: Ausbringungsorgan
- 3, 3₁, 3ₙ: (horizontales) Innenraumelement mit Gitter-, Sieb- oder Netzstruktur
- 4: Tropfen
- 5: Pumpensystem
- 6: Auffangbereich
- 7: (vertikales) Innenraumelement (Netz, Schnur, Band oder Kette)
- 8: Gitterfenster
- 9, 9', 9", 9"': Gittersteg
- 10, 10', 10", 10"': Gitterknoten

## Patentansprüche

1. Verfahren zur Produktion von Biomasse aus phototrophen oder mixotrophen Organismen oder Zellen, bei dem in einer Suspension enthaltene Organismen oder Zellen in einem Photobioreaktor unter Zufuhr von Energie in Form natürlichen oder künstlichen Lichts und von mindestens CO₂ als gasförmigen Nährstoff kultiviert werden und die Biomasse nach der Kultivierung der Organismen oder Zellen durch Abtrennung aus der Suspension geerntet wird, wobei die Suspension während der Kultivierung der Miroorganismen oder Zellen in dem Photobioreaktor in einem Umlauf geführt wird, indem die Suspension über mindestens ein Ausbringungsorgan in einem oberen Bereich eines Kulturraums des Photobioreaktors ausgebracht wird, die sich durch das Wirken der Schwerkraft vollziehende Abwärtsbewegung der Suspension in dem Kulturraum für eine intensive Exposition der Organismen oder Zellen mit in den Kulturraum eintretendem oder darin erzeugtem Licht und zur Exposition mit dem oder den gasförmigen Nährstoffen durch mindestens ein geeignetes in dem Kulturraum angeordnetes Innenraumelement verzögert wird und die am Boden des Kulturraums aufgefangene Suspension mittels eines Pumpsystems erneut dem mindestens einem Ausbringungsorgan zugeführt wird, **dadurch gekennzeichnet, dass** die Suspension an dafür ausgebildeten Strukturen des oder der Innenraumelemente des Kulturraums zur Ausbildung einer Vielzahl von Tropfen veranlasst wird, wobei die in Tropfen überführten Anteile der Suspension während des Passierens des Kulturraums jeweils zumindest einmalig einen folgende Stadien aufweisenden Tropfenzyklus durchlaufen
a. Bildung des Tropfens an der dafür im Kulturraum vorgesehen Struktur,
b. Vergrößerung des Tropfens bis auf eine maximale Größe, wobei die in dem Tropfen enthaltenen Organismen oder Zellen während der Vergrößerung des Tropfens dem Licht und dem oder den gasförmigen Nährstoffen ausgesetzt sind und sich vermehren,
c. Abtropfen des Tropfens in einen Auffangbereich am Boden des Kulturraums oder auf eine weitere Tropfen erzeugende Struktur eines Innenraumelements.

2. Verfahren nach Anspruch 1, **dadurch gekennzeichnet, dass** zur Produktion der Biomasse phototrophe oder mixotrophe Mikroalgen kultiviert werden.

3. Verfahren nach Anspruch 1 oder 2, **dadurch gekennzeichnet, dass** die Suspension durch eine entsprechende Ausbildung der Innenraumelemente und/oder eine entsprechende Steuerung ihrer Ausbringung über das mindestens eine Ausbringungsorgan zur Bildung überwiegend linsenförmiger Tropfen veranlasst wird.

4. Verfahren nach Anspruch 1 oder 2, **dadurch gekennzeichnet, dass** die Suspension durch eine entsprechende Gestaltung der Innenraumelemente zu einer nebelartigen Tropfenbildung veranlasst wird.

5. Verfahren nach einem der Ansprüche 1 bis 4, **dadurch gekennzeichnet, dass** an den entsprechenden Strukturen des Kulturraums ausgebildete Tropfen zur Ernte der Biomasse von diesen durch Schwingungseinwirkung oder stoßartige Erschütterungen gelöst werden und die sich am Boden des Kulturraums sammelnde Suspension mit den darin enthaltenen Organismen oder Zellen Mitteln zur Abtrennung der Organismen oder Zellen aus der Suspension zugeleitet wird.

6. Verfahren nach Anspruch 5, **dadurch gekennzeichnet, dass** die Tropfen von den Strukturen des Kulturraums mittels auf diese Strukturen einwirkenden Ultraschalls gelöst werden.

7. Verfahren nach einem der Ansprüche 1 bis 4, **dadurch gekennzeichnet, dass** an den entsprechenden Strukturen des Kulturraums ausgebildete Tropfen zur Ernte der Biomasse von diesen abgeblasen werden und die sich am Boden des Kulturraums sammelnde Suspension mit den darin enthaltenen Organismen oder Zellen Mitteln zur Abtrennung der Organismen oder Zellen aus der Suspension zugeleitet wird.

8. Photobioreaktor zur Kultivierung phototropher Organismen oder Zellen, mit einem Kulturraum (1), welcher von natürlichem Licht oder von außerhalb oder innerhalb des Kulturraums erzeugtem künstlichen Licht durchsetzt ist und in welchen mindestens CO₂ als gasförmiger Nährstoff eingebracht wird, mit mindestens einem Ausbringungsorgan (2), über welches die in einer Suspension enthaltenen Organismen oder Zellen zur Exposition mit dem Licht und dem oder den gasförmigen Nährstoffen in einem oberen Bereich des Kulturraums ausgebracht werden, mit mindestens einem im Kulturraum (1) angeordneten Innenraumelement (3, 3₁, 3ₙ, 7), durch welches oder welche die sich aufgrund des Wirkens der Schwerkraft vollziehende Abwärtsbewegung der Suspension in dem Kulturraum (1) verzögert wird und einem Pumpsystem (5), durch welches die am Boden des Kulturraums (1) aufgefangene Suspension zur Realisierung eines Umlaufs in dem Photobioreaktor erneut dem mindestens einem Ausbringungsorgan (2) zugeführt wird, **dadurch gekennzeichnet, dass** in dem Kulturraum (1) unterhalb des mindestens einen Ausbringungsorgans (2) mindestens ein Innenraumelement (3, 3₁, 3ₙ) mit einer sich horizontal erstreckenden Gitter-, Sieb- oder Netzstruktur angeordnet ist, durch welche die in den Kulturraum (1) eingebrachte Suspension mit den Organismen oder Zellen zur Ausbildung einer Vielzahl von Tropfen (4) veranlasst wird, die sich nach ihrer Entstehung vergrößern und jeweils nach dem Erreichen einer maximalen Tropfengröße in einen Auffangbereich (6) am Boden des Kulturraums oder auf eine weitere im Kulturraum (1) angeordnete Tropfen (4) erzeugende Struktur eines Innenraumelements (3, 3₁, 3ₙ) abtropfen.

9. Photobioreaktor nach Anspruche 8, **dadurch gekennzeichnet, dass** in dem Kulturraum (1) mehrere sich horizontal erstreckende Innenraumelemente (3, 3₁, 3ₙ) mit jeweils einer Gitter-, Sieb- oder Netzstruktur in einer Kaskade untereinander angeordnet sind.

10. Photobioreaktor nach Anspruch 8 oder 9, **dadurch gekennzeichnet, dass** die Gitter-, Sieb- oder Netzstruktur des mindestens einen horizontalen Innenraumelements (3, 3₁, 3ₙ) aus einem hydrophoben Material besteht.

11. Photobioreaktor nach einem der Ansprüche 8 bis 10, **dadurch gekennzeichnet, dass** die Gitter-, Sieb- oder Netzstruktur des mindestens einen horizontalen Innenraumelements (3, 3₁, 3ₙ) aus einem transparenten Material besteht.

12. Photobioreaktor nach einem der Ansprüche 8 bis 10, **dadurch gekennzeichnet, dass** die Gitter-, Sieb- oder Netzstruktur des mindestens einen horizontalen Innenraumelements (3, 3₁, 3ₙ) aus einem lichtleitenden Material besteht.

13. Photobioreaktor nach einem der Ansprüche 8 bis 12, **dadurch gekennzeichnet, dass** in dem Kulturraum (1) mehrere, sich von der horizontalen Gitter-, Sieb- oder Netzstruktur des einen oder des letzten Innenraumelements (3, 3₁, 3ₙ) vertikal nach unten erstreckende Netze, Schnüre, Bänder oder Ketten (7) angeordnet sind, über welche von der betreffenden horizontalen Gitter-, Sieb- oder Netzstruktur abtropfende Tropfen (4) der Suspension in Richtung des Auffangbereichs (6) des Kulturraums (1) nach unten laufen.

14. Photobioreaktor nach einem der Ansprüche 8 bis 13, **dadurch gekennzeichnet, dass** dieser einen Ultraschallsender aufweist, dessen Ultraschallschwingungen zum Lösen verbliebener Tropfen (4) auf die Gitter-, Sieb- oder Netzstruktur des mindestens einen Innenraumelements (3, 3₁, 3ₙ) einwirken.

15. Photobioreaktor nach einem der Ansprüche 8 bis 12, **dadurch gekennzeichnet, dass** dieser ein Gebläse zum Lösen an der Gitter-, Sieb- oder Netzstruktur des mindestens einen Innenraumelements (3, 3₁, 3ₙ) aufweist.

## Claims

1. A method for producing biomass composed of phototrophic or mixotrophic organisms or cells, in which organisms or cells contained in a suspension are cultivated in a photobioreactor with introduction of energy in the form of natural or artificial light and of at least CO₂ as a gaseous nutrient, and after cultivation of the organisms or cells, the biomass is harvested by separation from the suspension, whereby the suspension is circulated during the cultivation of the microorganisms or cells in the photobioreactor by introducing the suspension via at least one introduction organ in an upper region of a culturing space of the photobioreactor, the downward movement of the suspension in the culturing space that occurs due to the effect of gravity is slowed down by at least one suitable inner element disposed in the culturing space for an intensive exposure of the organisms or cells to light entering into the culturing space or produced therein and for exposure to the one or more gaseous nutrients, and the suspension collecting at the bottom of the culturing space is conducted repeatedly to the at least one introduction organ by means of a pumping system, is hereby **characterised in that** the suspension is inducted to the formation of a plurality of drops by structures of the one or more inner elements of the culturing space, which are formed for this purpose, whereby the parts of the suspension converted into drops during passage in the culturing space each pass through at least once a drop cycle having the following stages
a. formation of the drop on the structure provided for this in the culturing space,
b. enlargement of the drop up to a maximum size, whereby the organisms or cells contained in the drop are exposed to light and to the one or more gaseous nutrients during the enlargement of the drop and are multiplied,
c. dripping the drop down into a collection region at the bottom of the culturing space or onto another structure of an inner element that produces drops.

2. The method according to claim 1, further **characterised in that** phototrophic or mixotrophic microalgae are cultivated for the production of the biomass.

3. The method according to claim 1 or 2, further **characterised in that** the suspension is passed through an appropriate formation of the inner element and/or an appropriate control of its introduction via the at least one introduction organ for the formation of predominantly lens-shaped drops.

4. The method according to claim 1 or 2, further **characterised in that** the suspension is passed through an appropriate configuration of the inner element for a mist-like drop formation.

5. The method according to one of claims 1 to 4, further **characterised in that** drops formed on the corresponding structures of the culturing space are removed from these structures by vibration effect or shock-like vibrations for the harvesting of the biomass, and the suspension with the organisms or cells contained therein, which collects at the bottom of the culturing space, is then conducted to means for separating the organisms or cells from the suspension.

6. The method according to claim 5, further **characterised in that** the drops are removed from the structures of the culturing space by means of ultrasound acting on these structures.

7. The method according to one of claims 1 to 4, further **characterised in that** drops formed on the corresponding structures of the culturing space are blown off from these structures for the harvesting of the biomass, and the suspension with the organisms or cells contained therein, which collects at the bottom of the culturing space, is then conducted to means for separating the organisms or cells from the suspension.

8. A photobioreactor for cultivating phototrophic organisms or cells having a culturing space (1), through which passes natural light or artificial light produced outside or inside the culturing space and in which at least CO₂ is introduced as a gaseous nutrient, with at least one introduction organ (2), by means of which the organisms or cells contained in the suspension are introduced in an upper region of the culturing space for exposure to light and to the one or more gaseous nutrients, having at least one inner element (3, 3₁, 3ₙ, 7), disposed in culturing space (1), by means of which the downward movement of the suspension in culturing space (1), which occurs due to the effect of gravity, is delayed, and a pumping system (5), by means of which the suspension collecting at the bottom of culturing space (1) is conducted repeatedly to the at least one introduction organ (2) for providing circulation, is hereby **characterised in that** at least one inner element (3, 3₁, 3ₙ) with a grid, screen, or net structure extending horizontally is disposed in culturing space (1) underneath the at least one introduction organ (2), by means of which the suspension containing the organisms or cells, which has been introduced into culturing space (1), is inducted to the formation of a plurality of drops (4), which, after they have formed, increase in size and in each case, after reaching a maximum drop size, are dripped down into a collecting region (6) at the bottom of the culturing space or onto another structure of an inner element (3, 3₁, 3ₙ) that produces drops (4) and is disposed in culturing space (1).

9. The photobioreactor according to claim 8, further **characterised in that** several inner elements (3, 3₁, 3ₙ) that extend horizontally in culturing space (1), each having a grid, screen or net structure, are disposed in a cascade, one underneath the other.

10. The photobioreactor according to claim 8 or 9, further **characterised in that** the grid, sieve or net structure of the at least one horizontal inner element (3, 3₁, 3ₙ) is composed of a hydrophobic material.

11. The photobioreactor according to one of claims 8 to 10, further **characterised in that** the grid, sieve or net structure of the at least one horizontal inner element (3, 3₁, 3ₙ) is composed of a transparent material.

12. The photobioreactor according to one of claims 8 to 10, further **characterised in that** the grid, sieve or net structure of the at least one horizontal inner element (3, 3₁, 3ₙ) is composed of a light-conducting material.

13. The photobioreactor according to one of claims 8 to 12, further **characterised in that** several nets, cords, strips or chains (7) that extend vertically downward from the horizontal grid, sieve or net structure of one of the inner elements (3, 3₁, 3ₙ) or of the last inner element (3, 3₁, 3ₙ) are disposed in culturing space (1), by means of which drops (4) of the suspension dripping down from the respective horizontal grid, sieve or net structure run downward in the direction of collecting region (6) of culturing space (1).

14. The photobioreactor according to one of claims 8 to 13, further **characterised in that** it has an ultrasonic transmitter, the ultrasonic vibrations of which act to remove drops (4) remaining on the grid, screen or net structure of the at least one inner element (3, 3₁, 3ₙ).

15. The photobioreactor according to one of claims 8 to 12, further **characterised in that** it has a fan for removing [drops remaining] on the grid, screen or net structure of the at least one inner element (3, 3₁, 3ₙ).

## Revendications

1. Procédé de production de biomasse à partir d'organismes ou cellules phototrophes ou mixotrophes, dans lequel
des organismes ou cellules que contient une suspension sont cultivés dans un photobioréacteur auquel de l'énergie est apportée sous la forme de lumière naturelle ou artificielle et avec au moins du CO₂ comme nutriment gazeux,
la biomasse étant récoltée après la culture en séparant les organismes ou les cellules de la suspension,
la suspension étant mise en recirculation pendant la culture des microorganismes ou des cellules dans le photobioréacteur en extrayant la suspension par au moins un organe d'extraction situé dans une partie supérieure,
le déplacement de la suspension vers le bas de l'espace de culture sous l'action de la gravité étant ralenti par au moins un élément approprié d'espace intérieur disposé dans l'espace de culture en vue d'une exposition intensive des organismes ou cellules à la lumière qui pénètre dans l'espace de culture ou qui y est produite et en vue de leur exposition au nutriment ou aux nutriments gazeux,
la suspension reprise dans le fond de l'espace de culture étant de nouveau amenée à l'organe ou aux organes d'extraction au moyen d'un système de pompe, **caractérisé en ce que**
la suspension est admise sur des structures appropriées du ou des éléments d'espace intérieur de l'espace de culture en vue de former des gouttes, chacune des fractions de la suspension transformées en gouttes parcourant au moins une fois lors de la traversée de l'espace de culture un cycle de gouttes qui présente les stades suivants:
a. formation de la goutte sur la structure prévue à cet effet dans l'espace de culture,
b. croissance de la goutte jusqu'à une taille maximale, les organismes ou cellules que contiennent la goutte étant exposés pendant l'agrandissement de la goutte à la lumière et au nutriment ou aux nutriments gazeux et se multipliant,
c. chute de la goutte dans une zone de reprise située au fond de l'espace de culture ou sur une autre structure de formation de gouttes d'un élément d'espace intérieur.

2. Procédé selon la revendication 1, **caractérisé en ce que** des microalgues phototrophes ou mixotrophes sont cultivées pour produire de la biomasse.

3. Procédé selon les revendications 1 ou 2, **caractérisé en ce que** la suspension est transformée en gouttes principalement en forme de lentille grâce à une configuration appropriée des éléments d'espace intérieur et/ou à un contrôle approprié de leur amenée par le ou les organes d'extraction.

4. Procédé selon les revendications 1 ou 2, **caractérisé en ce que** la suspension est amenée à former des gouttes en forme de brouillard par une configuration appropriée des éléments d'espace intérieur.

5. Procédé selon l'une des revendications 1 à 4, **caractérisé en ce que** pour la récolte de la biomasse, les gouttes formées sur les structures appropriées de l'espace de culture sont libérées de ces structures par l'effet d'oscillations ou de brusques secousses et **en ce que** la suspension qui se rassemble à la base de l'espace de culture et qui contient les organismes ou cellules est amenée à des moyens permettant de séparer les organismes ou cellules de la suspension.

6. Procédé selon la revendication 5, **caractérisé en ce que** les gouttes sont libérées par les structures de l'espace de culture au moyen d'ultrasons qui agissent sur ces structures.

7. Procédé selon l'une des revendications 1 à 4, **caractérisé en ce que** pour la récolte de la biomasse, les gouttes formées sur les structures appropriées de l'espace de culture sont soufflées de ces structures et **en ce que** la suspension qui se rassemble à la base de l'espace de culture et qui contient les organismes ou cellules est amenée à des moyens permettant de séparer les organismes ou cellules de la suspension.

8. Photobioréacteur destiné à cultiver des organismes ou cellules phototrophes et présentant
un espace de culture (1) qui est traversé par de la lumière naturelle ou de la lumière artificielle produite à l'extérieur ou à l'intérieur de l'espace de culture et dans lequel au moins du CO₂ est apporté en tant que nutriment gazeux,
au moins un organe d'extraction (2) par lequel les organismes ou cellules que contient une suspension sont extraits dans une partie supérieure de l'espace de culture pour être exposés à la lumière et au nutriment ou aux nutriments gazeux,
au moins un élément (3, 3₁, 3ₙ, 7) d'espace intérieur disposé dans l'espace de culture (1) et par lequel ou lesquels le déplacement de la suspension vers le bas de l'espace de culture (1) qui s'établit sous l'action de la gravité est ralenti et
un système de pompage (5) par lequel la suspension reprise à la base de l'espace de culture (1) est de nouveau amenée à l'organe ou aux organes d'extraction (2) pour réaliser une recirculation dans le photobioréacteur,
**caractérisé en ce que**
au moins un élément (3, 3₁, 3ₙ) d'espace intérieur qui présente une structure en grille, en tamis ou en treillis s'étendant horizontalement est disposé dans l'espace de culture (1) en dessous du ou des organes d'extraction (2) et amène la suspension contenant les organismes ou cellules et amenée dans l'espace de culture (1) à former des gouttes (4) qui grandissent après être apparues et tombent, chacune après avoir atteint une taille maximale, dans une zone de reprise (6) située à la base de l'espace de culture ou sur une autre structure de formation de gouttes (4) d'un élément (3, 3₁, 3ₙ) d'espace intérieur situé dans l'espace de culture (1).

9. Photobioréacteur selon la revendication 8, **caractérisé en ce que** plusieurs éléments (3, 3₁, 3ₙ) d'espace intérieur qui présentent tous une structure en grille, en tamis ou en treillis sont disposés en cascade les uns en dessous des autres dans l'espace de culture (1).

10. Photobioréacteur selon les revendications 8 ou 9, **caractérisé en ce que** la structure en grille, en tamis ou en treillis du ou des éléments horizontaux (3, 3₁, 3ₙ) d'espace intérieur est constituée d'un matériau hydrophobe.

11. Photobioréacteur selon l'une des revendications 8 à 10, **caractérisé en ce que** la structure en grille, en tamis ou en treillis du ou des éléments horizontaux (3, 3₁, 3ₙ) d'espace intérieur est constituée d'un matériau transparent.

12. Photobioréacteur selon l'une des revendications 8 à 10, **caractérisé en ce que** la structure en grille, en tamis ou en treillis du ou des éléments horizontaux (3, 3₁, 3ₙ) d'espace intérieur est constituée d'un matériau conducteur de la lumière.

13. Photobioréacteur selon l'une des revendications 8 à 12, **caractérisé en ce que** plusieurs treillis, cordons, bandes ou chaînes (7) qui s'étendent verticalement vers le bas depuis la structure horizontale en grille en tamis ou en treillis d'un élément ou du dernier élément (3, 3₁, 3ₙ) d'espace intérieur et par lesquels les gouttes (4) de suspension qui tombent de la structure horizontale concernées en grille, en tamis ou en treillis s'écoulent vers le bas en direction de la zone de reprise (6) de l'espace de culture (1) sont disposés dans l'espace de culture (1).

14. Photobioréacteur selon l'une des revendications 8 à 13, **caractérisé en ce qu'**il présente un émetteur d'ultrasons dont les ultrasons agissent pour libérer les gouttes (4) encore présentes sur la structure en grille, en tamis ou en treillis du ou des éléments (3, 3₁, 3ₙ) d'espace intérieur.

15. Photobioréacteur selon l'une des revendications 8 à 12, **caractérisé en ce qu'**il présente un ventilateur de libération sur la structure en grille, en tamis ou en treillis du ou des éléments (3, 3₁, 3ₙ) d'espace intérieur.
